**Europäisches Patentamt**

**European Patent Office**

**Office européen des brevets**

(11) Numéro de publication: **0 309 300 B1**

(12) **FASCICULE DE BREVET EUROPEEN**

(45) Date de publication de fascicule du brevet: **17.06.92**

(51) Int. Cl.5: **C07C 227/18**, C07C 229/22

(21) Numéro de dépôt: **88400765.9**

(22) Date de dépôt: **29.03.88**

(54) **Procédé de préparation de sels de l'acide dimethylglycylgluconique.**

(30) Priorité: **21.09.87 PT 85759**

(43) Date de publication de la demande:
**29.03.89 Bulletin 89/13**

(45) Mention de la délivrance du brevet:
**17.06.92 Bulletin 92/25**

(84) Etats contractants désignés:
**AT BE CH DE ES FR GB GR IT LI LU NL SE**

(56) Documents cités:
**DE-A- 1 953 913**
**US-A- 2 710 876**
**US-A- 3 907 869**

**CHEMICAL ABSTRACTS, vol. 61, no. 7091h,
14 septembre 1964, Columbus, Ohio, US; &
SU-A-161 719**

(73) Titulaire: **TECNIMEDE-SOCIEDADE TECNICO
MEDICINAL, LTDA
Urb. Ouinta Nova Rua B, Lote 131-A
Sacavem(PT)**

(72) Inventeur: **Nascimento, José Maria, Jr.
urb. de Portela Lote 18-2. Dt.
Sacavém(PT)**
Inventeur: **Pinto de Carvalho Gonzaga Bronze,
Arlete
Rua Pereira Basto No. 7
Alfragide(PT)**

(74) Mandataire: **Gillard, Marie-Louise et al
Cabinet Beau de Loménie 55, Rue d'Amsterdam
F-75008 Paris(FR)**

Rank Xerox (UK) Business Services

# Description

La présente invention concerne, en général, un procédé pour la préparation des sels de l'acide pangamique ou acide diméthylglycylgluconique, et en particulier, du pangamate de calcium et du pangamate de magnésium, plus précisément appelés diméthylglycylgluconates, qui sont appropriés pour être utilisés en médecine humaine comme produits pharmaceutiques ayant des applications diverses.

Du point de vue chimique, l'acide pangamique est un ester de l'acide gluconique et de la diméthylglycine. L'acide et les sels respectifs, parfois aussi inexactement appelés vitamine $B_{15}$, et dont le nom plus approprié est diméthylglycylgluconates, sont capables d'accroître le métabolisme respiratoire au niveau des cellules et des tissus, notamment la phosphorylation oxydative, et d'éliminer quelques aspects des phénomènes et des conséquences de l'hypoxie. Ils ont également la capacité de promouvoir le métabolisme des lipides et d'avoir une activité désintoxicante.

Etant donné les activités plus haut mentionnées, cette substance et les préparations respectives sont indiquées pour soigner les formes diverses d'artériosclérose, y compris les formes chroniques d'insuffisance coronaire d'artériosclérose cérébrale, les altérations pulmonaires ou respiratoires, l'insuffisance cardio-respiratoire ainsi que les altérations hépatiques, les stades de lésions hépatiques évolutives et l'intoxication éthylique (alcoolisme).

Cette substance est aussi utile pour soigner certaines lésions dermatologiques (dermatoses prurigineuses, etc...).

Elle augmente la capacité de tolérer les corticostéroïdes sulfanilamides et d'autres substances éventuellement toxiques.

Ces pangamates sont utiles en médecine sportive comme facteur favorisant l'adaptation de l'organisme humain à une activité musculaire intense. Ils peuvent être administrés, à raison de un ou deux doses quotidiennes, la dose totale quotidienne étant de 0,2 à 0,4 g.

Quelques méthodes de production de l'acide pangamique ont déjà été divulguées, mais elles sont soit excessivement complexes et coûteuses pour être appliquées industriellement, soit de rentabilité excessivement réduite. Par ailleurs, elles sont mises en question relativement à la substance produite (Brevet US 2.710.876 - 1955 et critiques - J.A.M.A.).

Ainsi, par exemple, et en conformité avec le brevet susmentionné, l'estérification de l'acide gluconique avec du chlorhydrate de diméthylglycine a lieu en saturant une solution aqueuse avec de l'acide chlorhydrique gazeux, tout en la maintenant à la température ambiante pendant deux semaines, pendant lesquelles l'acide chlorhydrique et les mélanges sont éliminés du milieu réactionnel par dialyse à travers des membranes spéciales, et le produit dialysé contenant l'acide pangamique est neutralisé avec de la soude caustique et séché par lyophilisation. La rentabilité en produit final (pangamate de sodium) obtenue est d'environ 25 % de la valeur théorique et la préparation finale est fortement hygroscopique.

Selon le brevet portugais n° 79.359, on procède à l'élimination de l'acide sulfurique utilisé comme catalyseur par action du carbonate de calcium, ce qui fournit une suspension de carbonate et de sulfate de calcium, dont la filtration est difficile, contaminant le produit final avec des sulfates et rendant difficile l'obtention du pangamate de calcium sous la forme d'une poudre éparse.

La présente invention a pour objet une méthode industriellement appropriée pour produire l'acide pangamique ou ses sels respectifs, sous des formes moins hygroscopiques et moins contaminées par des sels minéraux.

Selon la présente invention, la réaction d'estérification de l'acide gluconique ou de ses sels respectifs avec le chlorhydrate de diméthylglycine est effectuée en milieu aqueux en présence de catalyseurs acides, tels que l'acide phosphorique ou l'acide sulfurique, à des températures variant entre 30° et 70° C, de préférence entre 30 et 50° C ; cette réaction est suivie de la concentration sous vide du produit de l'estérification jusqu'à ce que celui-là atteigne une consistance sirupeuse. On effectue alors l'élimination des acides minéraux, au moyen d'une amine aliphatique de masse moléculaire élevée, insoluble dans l'eau et on transforme l'acide pangamique en l'un de ses sels métalliques, par l'addition d'une quantité d'un carbonate métallique légèrement supérieure à la quantité stoechiométrique pour la neutralisation de l'acide pangamique, par exemple le carbonate de calcium ou le carbonate de magnésium.

La solution ainsi obtenue est concentrée à une température non supérieure à 50° C jusqu'à ce qu'elle atteigne une consistance de sirop et est précipitée en ajoutant de l'alcool aliphatique ayant un faible poids moléculaire, tel que, par exemple le méthanol ou l'éthanol.

Le produit final présente, avec n'importe quel catalyseur acide, un pourcentage élevé du produit de condensation qu'on peut identifier par chromatographie en couche mince ensemble avec les produits initiaux (acide gluconique et diméthylglycine) et dont la condensation est confirmée par le spectre infra-rouge où on peut observer une bande à 1 750 $cm^{-1}$, caractéristique de la fonction ester.

Cette méthode de préparation fournit un produit pulvérulent blanc ou légèrement jaunâtre (pangamate de calcium et de magnésium) insoluble dans l'alcool et qui, dû à son hygroscopicité, doit être conservé dans un récipient hermétique.

La méthode proposée présente les avantages suivants :

a) Réaction d'estérification rapide entre le chlorhydrate de diméthylglycine et les gluconates ;

b) Obtention d'une solution d'acide pangamique sans sels minéraux, ce qui fournit des produit finals ayant un taux en cendres très réduit.

c) Facilité de production d'autres sels d'acide pangamique différents.

d) Procédé de préparation plus simple que ceux qui sont divulgués dans l'art antérieur en ce qui concerne la purification du produit final.

e) Réalisation dans les conditions et avec des équipements conventionnels.

Les exemples qui suivent servent à illustrer la méthode selon l'invention.

## EXEMPLE 1 -

104 g de gluconate de magnésium ou 108 g de gluconate de calcium (0,5 mole) et 83,4 g de chlorhydrate de diméthylglycine (0,6 mole) sont dissous dans 200 ml d'eau, tout en chauffant légèrement. On ajoute ensuite 50 ml (90g) d'acide sulfurique concentré à la solution obtenue et on maintient le mélange sous agitation constante, à 40° C pendant 3 heures.

Ensuite l'eau est distillée sous vide jusqu'à ce qu'on obtienne un résidu visqueux. Cette masse est laissée au repos pendant 15 ou 18 heures à 30° C.

Pendant cette période, la masse subit une dilution. On élimine les acides minéraux par addition d'une solution de 800 g de trioctylamine dans 0,8 l d'éther de pétrole.

La phase aqueuse contenant l'acide pangamique est ajoutée à une quantité d'eau non supérieure à 200 ml afin de réduire la viscosité de la phase aqueuse de façon à permettre l'addition des carbonates.

On ajoute, en agitant le mélange, 70 g de carbonate de calcium ou 60 g de carbonate de magnésium jusqu'à ce que l'effervescence disparaisse.

On élimine par filtration le léger résidu insoluble.

La solution aqueuse est concentrée sous vide, à une température ne dépassant pas 45° C jusqu'à 70 % de matière solide.

On ajoute lentement, en agitant tout le temps, environ 1 litre de méthanol ou d'éthanol.

Le produit solide est obtenu par filtration et séché avec un dessiccateur sous vide.

Le contrôle du produit de la réaction est fait par chromatographie sur plaque de silice, l'éluant étant le mélange butanol-acide acétique-eau (50:25:25) et la visualisation est faite avec une solution de permanganate de potassium à 0,5 % en hydroxyde de sodium normal.

## EXEMPLE 2 -

104 g de gluconate de magnésium ou 108 g de gluconate de calcium (0,5 mole) et 83,4 g de chlorhydrate de diméthylglycine (0,6 mole) sont dissous dans 200 ml d'eau tout en chauffant légèrement. On ajoute 60 ml d'acide phosphorique (85 %) à la solution obtenue et le mélange est maintenu sous agitation constante pendant 3 heures, à 40° C.

Ensuite l'eau est distillée sous vide, jusqu'à ce qu'on obtienne un résidu visqueux. La masse réactionnelle est laissée au repos pendant 15 à 18 heures à 30° C.

Pendant cette période, la masse subit une dilution. Les acides minéraux sont éliminés par addition d'une solution de 800 g de trioctylamine dans 0,8 l d'éther de pétrole.

A la phase aqueuse contenant l'acide pangamique, on ajoute une quantité d'eau non supérieure à 200 ml, pour réduire la viscosité de la phase aqueuse de façon à permettre l'addition des carbonates.

On ajoute, en agitant le mélange, 70 g de carbonate de calcium ou 60 g de carbonate de magnésium jusqu'à ce que disparaisse l'effervescence.

Le léger résidu insoluble est éliminé par filtration.

La solution aqueuse est concentrée sous vide à une température non supérieure à 45° C jusqu'à 70 % de matière solide.

On ajoute lentement, tout en agitant, environ 1 litre de méthanol ou d'éthanol.

Le produit solide est obtenu par filtration et séché dans un dessiccateur sous vide.

Le contrôle du produit de la réaction est fait par chromatographie sur plaque de silice, l'éluant étant le mélange butanol-acide acétique-eau (50:25:25) et la visualisation est faite avec une solution de permanganate de potassium à 0,5 F% en hydroxyde de sodium normal.

## Revendications

1. Procédé de préparation de sels de l'acide pangamique, caractérisé en ce qu'on effectue l'estérification de l'acide gluconique ou de ses sels de calcium ou de magnésium respectifs avec du chlorhydrate de diméthylglycine en présence d'acide sulfurique ou d'acide phosphorique, en milieu aqueux, à une température variant entre 30° et 70° C, suivie de l'élimination des acides minéraux du milieu réactionnel par action d'une amine aliphatique insoluble dans l'eau, et de la transformation de l'acide en l'un de ses sels métalliques par addition d'un carbonate métallique.

2. Procédé selon la revendication 1, caractérisé en ce que l'amine est la trioctylamine.

3. Procédé selon la revendication 1, caractérisé en ce que la quantité de carbonate ajoutée est à peine légèrement supérieure à la quantité stoechiométrique.

4. Procédé selon l'une quelconque des revendications 1 à 3, caractérisé en ce que la température est comprise entre 30 et 50°C.

5. Procédé selon l'une quelconque des revendications 1 à 4, caractérisé en ce que le carbonate est le carbonate de calcium ou le carbonate de magnésium.

## Claims

1. Process for the preparation of salts of pangamic acid, characterized in that esterification of gluconic acid or its calcium or magnesium salts respectively is carried out with dimethylglycine hydrochloride in the presence of sulphuric acid or phosphoric acid, in an agueous medium, at a temperature varying between 30° and 70°C, followed by the removal of the inorganic acids from the reaction medium by the action of an aliphatic amine which is insoluble in water, and the conversion of the acid to one of its metal salts by the addition of a metal carbonate.

2. Process according to Claim 1, characterized in that the amine is trioctylamine.

3. Process according to Claim 1, characterized in that the quantity of carbonate added is very slightly greater than the stoichiometric quantity.

4. Process according to any one of Claims 1 to 3, characterized in that the temperature is between 30 and 50°C.

5. Process according to any one of Claims 1 to 4, characterized in that the carbonate is calcium carbonate or magnesium carbonate.

## Patentansprüche

1. Verfahren zur Herstellung von Salzen der Pangamsäure, **gekennzeichnet durch**
Veresterung der Gluconsäure oder ihrer Calcium- oder Magnesiumsalze mit Dimethylglycin-hydrochlorid in Gegenwart von Schwefelsäure oder Phosphorsäure in wäßrigem Medium bei einer Temperatur von 30 bis 70 °C und
Entfernung der anorganischen Säuren aus dem Reaktionsmedium durch Umsetzung mit einem in Wasser unlöslichen aliphatischen Amin und Umwandlung der Säure in eines ihrer Metallsalze durch Zugabe eines Metallcarbonats.

2. Verfahren nach Anspruch 1, gekennzeichnet durch Verwendung von Trioctylamin als Amin.

3. Verfahren nach Anspruch 1, gekennzeichnet durch Verwendung einer zugesetzten Menge an Carbonat, die kaum höher als die stöchiometrische Menge ist.

4. Verfahren nach einem der Ansprüche 1 bis 3, gekennzeichnet durch Anwendung einer Temperatur von 30 bis 50 °C.

5. Verfahren nach einem der Ansprüche 1 bis 4, gekennzeichnet durch Verwendung von Calcium- oder Magnesiumcarbonat als Carbonat.